# FASCICULE DE BREVET EUROPEEN

(11) **EP 0 840 720 B1**
(45) Date de publication et mention de la délivrance du brevet: **10.09.2003**
(21) Numéro de dépôt: 96924964.8
(22) Date de dépôt: 10.07.1996
(51) Int. Cl.: C07C 67/307, A61K 31/23, C07C 69/63

(54) **PRODEDE POUR OBTENIR ACIDES GRAS IODES ET LEURS DERIVES PAR IODOHYDRINATION, FAISANT INTERVENIR DES DERIVES ALKYLSILYLES AVEC DES IODURES ALCALINS ET LEURS ACTIVITES PHARMACOLOGIQUES**
VERFAHREN ZUR HERSTELLUNG JODIERTER FETTSÄUREN UND IHRER DERIVATE DURCH JODWASSERSTOFFANLAGERUNG UNTER ANWENDUNG VON ALKYLSILYLDERIVATEN MIT ALKALIJODIDEN UND DEREN PHARMAKOLOGISCHEN AKTIVITÄTEN
PROCESS FOR MAKING IODINATED FATTY ACIDS AND DERIVATIVES THEREOF BY IODOHYDRINATION USING ALKYLSILYLATED DERIVATIVES AND ALKALINE IODIDES AND PHARMACOLOGICAL ACTIVITIES THEREOF

(30) Priorité: 11.07.1995 FR 9508582
(43) Date de publication de la demande: 13.05.1998
(73) Titulaire: Jung, Louis, 67200 Strasbourg (FR); Ingenbleek, Yves, 67000 Strasbourg (FR)
(72) Inventeur: Jung, Louis, 67200 Strasbourg (FR); Ingenbleek, Yves, 67000 Strasbourg (FR)
(74) Mandataire: Nuss, Pierre
(86) Numéro de dépôt international: FR9601075
(87) Numéro de publication internationale: WO97003038

(56) Documents cités:
- DE-A- 3 513 323
- DATABASE WPI Week 7847 Derwent Publications Ltd., London, GB; AN 78-84825A XP002006541 & JP,A,53 119 817 (SHINROIHI KK) , 19 Octobre 1978

## Description

L'invention a pour objet des esters d'acides gras iodés et des acides gras iodés et leurs dérivés, obtenus par iodohydrination faisant intervenir des dérivés alkylsilylés avec des iodures alcalins utilisés dans le traitement du goitre endémique et les compositions pharmaceutiques les renfermant. Il peuvent également être utilisés comme véhicule de chimioembolisation. Le goitre endémique est une maladie carentielle qui constitue un des plus graves problèmes de santé publique auxquels l'Organisation Mondiale de la Santé (OMS) soit confronté. Selon les indications officielles de l'OMS, un milliard d'individus, soit 20 % de la population mondiale est touché par le déficit iodé, principalement dans les pays en voie de développement (HETZEL B.S., POTTER B.J. and DULBERG E.M. The iodine deficiency disorders : nature, pathogenesis and epidemiology. World Rev. Nutr. Diet. 62 : 59-119 (1990)). La quasi totalité de ces pays est concernée avec des degrés de prévalence très variables. Dans les régions les plus sévèrement affectées, on peut dénombrer jusqu'à 80 % de sujets souffrant de dysfonctionnements thyroïdiens : ceux-ci se traduisent par l'apparition d'une hypertrophie glandulaire inesthétique pouvant évoluer vers une hypothyroïdie secondaire avec troubles neurologiques. Les adolescentes et les femmes en âge de procréer sont les plus exposées et peuvent donner naissance à une proportion élevée atteignant jusqu'à 10 % des nouveaux-nés souffrant d'une forme particulière de débilité mentale irréversible, appelée crétinisme endémique, et considérée comme la complication médicale et sociale la plus redoutable.
Il est universellement reconnu que le déficit alimentaire en iode est la cause première et largement prédominante de ce fléau nutritionnel. La nature géologique et l'environnement géographique apparaissent ainsi comme les déterminants principaux de cette situation, bien que certains facteurs alimentaires soient incriminés comme causes secondaires aggravantes. La thérapeutique appropriée consistera donc à supplémenter les populations déficientes par des apports iodés couvrant les besoins physiologiques.

Théoriquement, cet objectif est aisé à atteindre. Cependant, l'expérience des dernières décennies a montré que les véhicules traditionnels de supplémentation iodée (eau potable, sel de cuisine, farines boulangères) rencontrent dans les régions (sub)-tropicales des difficultés majeures de diffusion, liées aux conditions de transport, de conservation, de consommation. En effet, l'iode est administré sous forme d'iodure ou d'iodate de sodium ou de potassium, libérant l'halogène sous une forme non stockable dans les tissus adipeux. Une prise quotidienne du véhicule iodé est donc nécessaire pendant des années. Les bénéfices nutritionnels sont irréguliers et lents à obtenir, et cette forme de supplémentation ne permet pas de remédier rapidement aux problèmes urgents rencontrés dans les régions de haute prévalence.

Il existe également une huile iodée à effet-retard, appelée Lipiodol® (Société Guerbet) et qui, administrée sous une dose orale ou parentérale annuelle unique, a démontré des effets préventifs et thérapeutiques anti-goitreux. Cette huile iodée, conçue comme produit de contraste en radiologie, est fabriquée à partir d'huile d'oeillette (Somnifer papaverum) rare et relativement chère. Bien que la bonne tolérance et les vertus curatives du Lipiodol® soient connues depuis longtemps, ce produit ne s'est pas imposé comme outil d'éradication massive en raison d'un coût relativement élevé en regard des immenses besoins du Tiers-Monde.

La présente invention vise à supprimer cet handicap : il est proposé un nouveau médicament iodé, formé d'esters d'acides gras iodés ou acides gras iodés et leurs dérivés, de pureté pharmaceutique, stables, dépourvus d'impuretés toxiques, totalement iodés, ne présentant plus de doubles liaisons, obtenus par action d'un réactif alkylsilylé et d'un iodure alcalin sur des esters d'acides gras ou des acides gras et présentant des propriétés thérapeutiques.

Par exemple à partir d'esters méthyliques d'acides gras iodés obtenus par une synthèse originale à partir d'huile de colza (Brassica campestris) utilisés comme carburant biologique pour les moteurs de voitures d'un prix de revient très faible, il est possible de fabriquer des esters d'acides gras iodés de faible coût permettant d'envisager des campagnes de masse. Le nouveau produit assure une meilleure biodisponibilité et un effet thérapeutique prolongé car l'iode est fixé sur les trois acides gras (acide oléique n-9, acide linoléique n-6 et acide α-linolénique n-3) - dont les deux derniers sont essentiels - précurseurs des trois principales voies métaboliques des lipides. En vue de prévenir les risques de contamination virale par voie sanguine (hépatites B et C, VIH) l'administration du produit selon l'invention iodé est proposée exclusivement par voie orale.

Plusieurs méthodes ont déjà été décrites pour la transformation d'acides gras insaturés ou d'esters d'acides gras insaturés en dérivés saturés iodés.

L'acide oléique peut subir une hydrobromation suivie d'une substitution nucléophile par l'iodure de potassium après action de l'acide bromhydrique. (J.F. LANE and H.W. HEINE ; On cyclic Intermediates in Substitution Reactions. I. The Alkaline Hydrolysis of Some Aliphatic Bromoacids. J. Am. Chem. Soc. 1951, 73, 1348 -1350). L'huile d'olive refroidie au voisinage du point de prise en masse est saturée directement d'acide iodhydrique. D'autres acides gras ont été envisagés. (A. GUERBET, A. GIBAUD, G. TILLY, R. JOUSSOT, V. LOTH et M. GUERBET ; Monoiodostéarate d'éthyle. Préparation et caractères analytiques. Ann. pharm. fr., 1965, 23, N°11, 663-671). L'iodation directe par l'acide iodhydrique est également réalisée en utilisant des substances déshydratantes tels les acides polyphosphoriques, le pentoxyde de phosphore (W. KUHN, H. HARTNER, F. SCHINDLER, I. SANDNER et K. HERING ; brevet allemand P 35 13 322.6/C 07 C 69/62, 1985). L'hydroiodation peut également être réalisée par de l'iode en présence d'alumine générant de l'acide iodhydrique (L J. STEWART, D. GRAY, R. M. PAGNI and G. W. KABALKA ; A Convenient method for the addition of HI to unsaturated hydrocarbons using I₂ on Al₂O₃, Tetrahedron Lett, 1987, Vol. 28, N° 39, 4497-4498). L'hydroiodation a également été réalisée au moyen du complexe bore-N,N-diéthylamine faisant intervenir le triiodure de bore. (CH. KISHAN REDDY and M. PERIASAMY ; A new, simple procedure for the generation and addition of HI to alkenes and alkynes using BI₃ : N,N-diethylanilline complex and acetic acid. Tetrahedron Lett., 1990, Vol. 31, N°13, 1919-1920). L'hydroiodation par l'iodure de potassium dans l'acide orthophosphorique peut également être envisagée (Organic. Synthesis, vol 9, 66).

On connait également le document JP-A-53119817 qui divulgue une synthèse du tri-(2-iodohexadécanoate) de glycéryle en deux étapes.

Dans un premier temps, on fait réagir du chlorure de 2-bromopalmitoyle sur de la glycérine dans un solvant benzène/pyridine exempt d'eau, pour obtenir, après purification 36% de tri-(2-bromohexadécanoate) de glycéryle.

Puis on fait réagir ce dernier avec NaI dans de l'acétone pour donner, après traitement au Na₂S₂O₃, purification, séchage etc., 74% de tri-(2-iodohexadécanoate) de glycéryle.

Comme le précise, entre autres, le brevet allemand N° DE 3513 323. du 13.4.1985 de W. KUHN et coll., les méthodes de préparation de composés iodés conduisent à des produits contenant des impuretés toxiques, des produits peu stables à l'air et à la lumière lors de leur préparation et de leur conservation. La présente méthode de préparation conduit en particulier à un produit aux différents constituants ne présentant plus aucune double liaison, source d'instabilité en particulier en présence de dérivés d'oxydation et de radicaux libres. Les esters d'acides iodés gras préparés par iodohydrination faisant intervenir des dérivés alkylsilylés et un iodure alcalin, objet de l'invention, se présentent sous forme d'un liquide huileux, fluide, jaune clair, stable, pharmaceutiquement pur, d'un prix de revient relativement faible.

Les esters d'acides gras utilisés pour la préparation des dérivés iodés faisant intervenir un composé alkylsilylé et un iodure alcalin peuvent par exemple être des triglycérides d'acides gras provenant d'huiles végétales, du type huile de colza, huile d'oeillette, huile de soja, huile de carthame, huile d'arachides, huile de pépins de raisin, huile de tournesol, huile de lin, huile de maïs, huile d'olive, huile de sésame, huile de germes de blé, huile de noix de coco, huile de palme et huiles d'origine animale.

Les esters d'acides gras utilisés peuvent également être, des mélanges d'esters méthyliques ou d'esters éthyliques d'acides gras provenant d'huiles végétales, du type huile de colza, huile d'oeillette, huile de soja, huile de carthame, huile d'arachides, huile de pépins de raisin, huile de tournesol, huile de lin, huile de maïs, huile d'olive, huile de sésame, huile de germe de blé, huile de noix de coco, huile de palme et huiles d'origine animale.

Utiliser une matière première pour la synthèse des esters méthyliques d'acides gras provenant de l'huile de colza, utilisés comme carburant biologique pour moteur de voitures d'un prix de revient faible est particulièrement intéressante pour des productions importantes de médicaments.

Les acides gras du type acide oléique, acide linoléique, acide α-linolénique, acide érucique, acide arachidonique, acide ricinolique peuvent également être utilisés comme matières premières.

Le procédé pour l'obtention d'esters d'acides gras iodés ou d'acides gras iodés consiste à faire réagir un iodure alcalin avec un halogénurealkylsilylé en milieu organique donnant naissance en présence d'eau à de l'acide iodhydrique in situ réagissant soit avec l'ester ou les esters d'acides gras, soit avec les acides gras.

L'obtention d'esters d'acides gras iodés ou d'acides gras iodés consiste par exemple à faire réagir de l'iodure de sodium sur du chlorure de triméthylsilyle ou triméthylchlorosilane dans de l'acétonitrile suivie de l'action de l'eau et d'une addition soit d'esters d'acides gras insaturés, soit d'acides gras insaturés.

L'iodohydrination d'esters d'acides gras peut se faire selon la réaction suivante :

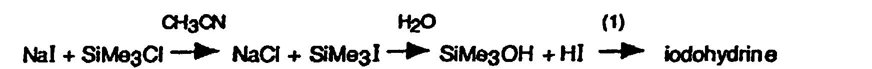

### (1) esters d'acides gras ou acides gras

Le mode opératoire par exemple pour des esters insaturés d'acides gras est le suivant : à une solution de 107,1 g d'iodure de sodium dans 550 ml d'acétonitrile, on ajoute sous atmosphère d'azote et à 0°C, 89,25 ml de triméthylchlorosilane(ou chlorure de triméthylsilyle). Après addition totale du triméthylchlorosilane, on ajoute au goutte à goutte 6,65 ml d'eau. Ensuite est ajoutée une solution de 70 g d'esters méthyliques ou éthyliques insaturés d'acides gras d'huile de colza. Après 24 heures de réaction sous agitation, la réaction est ensuite stoppée avec 700 ml d'eau. L'extraction est réalisée avec de l'éther. La phase organique est lavée plusieurs fois avec une solution de thiosulfate de sodium à 10 pour cent puis plusieurs fois à l'eau. Elle est séchée sur sulfate de sodium anhydre et l'éther est évaporé à une température de 90°C sous 2133 Pa (16 mm de Hg)afin d'éliminer les restes d'éther de silice. Le résidu est brun. Les esters d'acides gras iodés, dissous dans l'éther, sont ensuite décolorés sur du charbon puis filtrés sur de l'alumine afin d'éliminer les peroxydes. L'éther est évaporé et les traces de solvant sont éliminées à la pompe à palette. Le mélange d'esters d'acides gras iodés obtenus est jaune doré et présente une bonne fluidité. Ce mélange peut être caractérisé par spectrométrie 1H RMN et 13C RMN. Cette méthode de synthèse d'esters d'acides gras iodés a été réalisée sur des quantités plus importantes. D'autres solvants non miscibles à l'eau peuvent être utilisés. L'insolubilité du chlorure de sodium dans l'acétonitrile permet le déplacement total de la réaction vers la formation d'iodure de triméthylsilyle. Cette réaction est exothermique et permet la génération d'acide iodhydrique et d'hydroxyle de triméthylsilyle. Le produit secondaire SiMe₃OH est éliminé après la réaction d'iodohydrination par simple évaporation sous pression réduite et lavage à l'eau. La réaction d'iodohydrination est suivie visuellement par une décoloration de la solution. Le thiosulfate de sodium permet l'élimination de l'iode présent sous forme oxydée. Pour le produit final plus aucun signal en RMN du proton et en RMN carbone 13 ne correspond à un système éthylénique. D'autre part aucun produit de dégradation n'apparaît en RMN du proton.

**Caractéristiques des esters des acides gras Iodés de l'huile de colza :**
Résonance Magnétique Nucléaire du proton (RMN¹H) et du carbone 13 (RMN¹³C). Un spectre RMN1H des esters des acides gras iodés de l'huile de colza permet, par comparaison avec le spectre des esters des acides gras non iodés de l'huile de colza, de vérifier la disparition des protons éthyléniques. De plus, cela permet de contrôler l'absence de traces de diéthyléther solvant et d'éther de silice. Par ailleurs, un spectre RMN¹³C permet de vérifier la disparition des carbones portant les insaturations.
Les spectres RMN¹H obtenus pour plusieurs productions d'esters éthyliques d'acides gras iodés d'huile de colza sont identiques. Aucun spectre ne révèle la présence de diéthyléther ou d'éther de silice.
   - Spectre RMN¹H des esters éthyliques d'acides gras iodés [RMN¹H (CDCl₃) sur 200 MHz] : 0,89 ppm (3H, m,CH₃), 1,20-2,00 ppm (m, CH₂ des chaînes et CH₃CH₂O), 2,3 ppm (2H, t, CH₂-COOEt), 4,2 ppm (m, CHI, CH₂-OOC)
   - Spectre RMN¹³C des esters éthyliques d'acides gras iodés : RMN¹³C (CDCl₃) sur 200 MHz] : 14 ppm (CH₃ en bout de chaîne et CH₃CH₂O), 28-29 ppm (CHI), 22-24,30-31,34 ppm (CH₂ des chaînes), 39-41 ppm (CH₂-CHI), 60 ppm (CH₂-O), 173 ppm (COO).
      Le spectre d'absorption dans l'infrarouge (NaCl) présente les bandes caractéristiques suivantes : γ (C=O ester) à 1740 cm⁻¹ ; γ (CH saturé) à 2850 cm⁻¹, γ (CH saturé) à 2950 cm⁻¹
   - Spectre RMN¹H des esters méthyliques d'acides gras iodés [RMN¹H (CDCl₃) sur 200 MHz] : 0,89 ppm (3H, m, CH₃), 1,25 - 1,30 ppm (m, CH₂ des chaînes), 1,60 - 1,80 ppm (m, CH₂) 2,3 ppm (2H, t, CH₂-COO), 3,66 ppm (3H,s, CH₃-OOC), 4,12 ppm (m, CHI).
spectre RMN¹³C des esters méthyliques d'acides gras iodés [RMN¹³C (CDCl₃) sur 200 MHz] : 14 ppm (CH₃ en bout de chaîne), 28-29 ppm (CHI), 22 - 24, 31 - 34 ppm (CH₂ des chaînes), 38-41 ppm (CH₂-CHI), 51 ppm (CH₃-O), 173 ppm (COO).
**Stabilité** : Les produits iodés étant en général des substances instables, il est nécessaire de s'assurer de la stabilité des esters d'acides gras iodés ou/et des acides gras iodés. L'étude de stabilité est réalisée par RMN1H, par chromatographie sur couche mince et par un dosage de l'iode fixé sur les esters d'acides gras ou sur les acides gras.

Des spectres RMN1H ont été réalisés après 2 mois, 3 mois et 8 mois de conservation à température 20-22°C à l'abri de la lumière. Ils sont tous les trois identiques au spectre obtenu lors de la fabrication.

La chromatographie sur couche mince (plaque gel de silice GF 254-phase mobile : diéthyléther/hexane 1 : 20 - examen sous lumière ultraviolette à 254 nm et après pulvérisation d'une solution d'acide phosphomolybdique R à 10 pour cent *m*/*V* dans l'alcool et chauffage de la plaque à 120°C pendant 5 min) donne des taches observées identiques quant à leurs intensités et à leurs positions pour le produit fraîchement préparé et ceux conservés durant 8 mois.

Stabilité des esters d'acides gras iodés de l'huile de colza après emploi en thérapeutique sur le terrain : le mélange des esters d'acides gras iodés de l'huile de colza a fait l'objet d'une étude sur l'homme en Afrique, dans une zone de goitre endémique. Lors de cette étude, les esters iodés ont été soumis à des conditions extrêmes (transport, exposition pendant plusieurs heures à la lumière et à des températures de l'ordre de 45 °C). Au bout de deux semaines d'études, les esters d'acides gras iodés ont été analysés : le spectre RMN1H s'est avéré identique à celui d'une huile fraîchement synthétisée et la chromatographie sur couche mince donne des spots identiques à ceux des esters d'acides gras iodés fraîchement synthétisés, et ne révèle aucun produit de dégradation.

**Essai de tolérance chez le rat :** avant d'administrer le mélange d'esters d'acides gras iodés de l'huile de colza à des sujets goitreux, un essai de tolérance de mélange d'esters iodés est effectué sur des rats mâles adultes d'environ 300 g.

Chaque groupe test est constitué de 5 rats qui reçoivent chacun une administration orale de 0,5 ml de mélange d'esters d'acides gras iodés d'huile de colza.

Les rats sont gardés en observation. Au bout d'une semaine, le comportement et l'état général des rats de chaque groupe test sont normaux et identiques à ceux du groupe témoin.

Les produits iodés préparés selon la méthode décrite précédemment sont utilisés comme médicaments par voie générale, par exemple comme anti-goitreux à l'état pur ou en combinaison avec des excipients appropriés sous forme de liquide buvable ou ingérable, de gélules ou d'ampoules par exemple.

Ces produits peuvent être utilisés comme médicaments par voie générale ou par voie vasculaire locale dans le traitement de certains cancers par chimioembolisation consistant à administrer un anticancéreux émulsionné dans des esters d'acides gras iodés qui jouent le rôle de vecteurs pour les cellules lipophiles tumorales.

## Revendications

1. Procédé pour l'obtention d'au moins un acide gras iodé ou d'au moins un ester d'acide gras iodé ou de leurs dérivés iodés de pureté pharmaceutique, stable(s) et dépourvu(s) d'impuretés toxiques, **caractérisé en ce qu'**il consiste à faire réagir un iodure alcalin avec un réactif alkylsilylé en milieu organique donnant naissance, en présence d'eau, à de l'acide iodhydrique in situ réagissant avec le ou les acide(s) gras, le ou les ester(s) d'acide(s) gras ou avec leurs dérivés, de manière à ce que toutes les doubles liaisons initialement présentes dans le ou les acide(s) gras ou ester(s) d'acide(s) gras ou leurs dérivés soient saturées en iode à raison d'une molécule d'acide iodhydrique par double liaison.

2. Procédé selon la revendication 1, **caractérisé en ce que** l'iodure alcalin est l'iodure de sodium.

3. Procédé selon la revendication 1 ou 2, **caractérisé en ce que** le réactif alkylsilylé est un halogénure alkylsilylé.

4. Procédé selon la revendication 3, **caractérisé en ce que** l'halogénure alkylsilylé est du chlorure de triméthylsilyle ou triméthylchlorosilane.

5. Procédé selon l'une quelconque des revendications précédentes, **caractérisé en ce que** le milieu organique comporte de l'acétonitrile.

6. Procédé selon l'une quelconque des revendications précédentes, **caractérisé en ce que** le ou les ester(s) d'acide(s) gras consiste(nt) en un/des triglycéride(s) d'acides gras provenant d'huile(s) végétale(s) choisie(s) dans le groupe formé par l'huile de colza, l'huile d'oeillette, l'huile de soja, l'huile de carthame, l'huile d'arachides, l'huile de pépins de raisin, l'huile de tournesol, l'huile de lin, l'huile de maïs, l'huile d'olive, l'huile de sésame, l'huile de germes de blé, l'huile de noix de coco, l'huile de palme et les huiles d'origine animale.

7. Procédé selon l'une des revendications 1 à 5, **caractérisé en ce que** le ou les ester(s) d'acide(s) gras consiste(nt) en un/des ester(s) méthylique(s) d'acide(s) gras provenant d'huile(s) végétale(s) choisie(s) dans le groupe formé par l'huile de colza, l'huile d'oeillette, l'huile de soja, l'huile de carthame, l'huile d'arachides, l'huile de pépins de raisin, l'huile de tournesol, l'huile de lin, l'huile de maïs, l'huile d'olive, l'huile de sésame, l'huile de germes de blé, l'huile de noix de coco, l'huile de palme et les huiles d'origine animale.

8. Procédé selon la revendication 7, **caractérisé en ce que** le ou les ester(s) d'acide(s) gras consiste(nt) en un ou des ester(s) méthylique(s) d'acide(s) gras provenant de l'huile de colza, utilisé(s) comme carburant biologique pour moteurs de voitures.

9. Procédé selon l'une quelconque des revendication de 1 à 5, **caractérisé en ce que** le ou les ester(s) d'acide(s) gras consiste(nt) en un/des ester(s) éthylique(s) d'acide(s) gras provenant d'huile(s) végétale(s) choisie(s) dans le groupe formé par l'huile de colza, l'huile d'oeillette, l'huile de soja, l'huile de carthame, l'huile d'arachides, l'huile de pépins de raisin, l'huile de tournesol, l'huile de lin, l'huile de maïs, l'huile d'olive, l'huile de sésame, l'huile de germes de blé, l'huile de noix de coco, l'huile de palme et les huiles d'origine animale.

10. Procédé selon l'une quelconque des revendications de 1 à 5, **caractérisé en ce que** le ou les acide(s) gras est/sont choisi(s) dans le groupe formé par l'acide oléique, l'acide linoléique, l'acide α-linolénique, l'acide érucique, l'acide arachidonique et l'acide ricinoléique.

11. Acide(s) gras iodé(s), ester(s) d'acide(s) gras iodé(s) ou leurs dérivés iodés obtenu(s) par le procédé selon l'une quelconque des revendications 1 à 10, ne contenant aucune double liaison, pour la fabrication d'un médicament destiné au traitement de certains cancers par chimioemibolisation.

12. Acide(s) gras iodé(s), ester(s) d'acide(s) gras iodé(s) ou leurs dérivés iodés obtenu(s) par le procédé selon l'une quelconque des revendications 1 à 10, ne contenant aucune double liaison, pour la fabrication d'un anticancéreux émulsionné dans le ou lesdits acide(s) gras iodé(s), ester(s) d'acide(s) gras iodé(s) ou leurs dérivés iodés, ce ou ces derniers agissant comme vecteur(s) en vue d'atteindre les cellules tumorales lipophiles.

## Patentansprüche

1. Verfahren zur Gewinnung von mindestens einer jodhaltigen Fettsäure oder mindestens einem jodhaltigen Fettsäureester oder ihrer jodhaltigen Derivate von pharmazeutischer Reinheit, die stabil ist (sind) und keine toxischen Verunreinigungen aufweist(en),
**dadurch gekennzeichnet,**
**dass** es darin besteht, ein alkalisches Jodid mit einem alkylsilylierten Reagens in einem organischen Medium zur Reaktion zu bringen, wodurch im Beisein von Wasser Jodwasserstoffsäure entsteht, die in situ mit der oder den Fettsäure(n), dem oder den Fettsäureester(n) oder ihren Derivaten reagiert; so daß alle ursprünglich in der bzw. den Fettsäure(n) oder Fettsäureester(n) oder ihren Derivaten vorhandenen Doppelbindungen mit Jod in einer Menge von einem Molekül Jodwasserstoffsäure durch Doppelbindung gesättigt werden.

2. Verfahren nach Anspruch 1,
**dadurch gekennzeichnet,**
**dass** das alkalische Jodid ein Natriumjodid ist.

3. Verfahren nach Anspruch 1 oder 2,
**dadurch gekennzeichnet,**
**dass** das alkylsilylierte Reagens ein alkylsilyliertes Halogenid ist.

4. Verfahren nach Anspruch 3,
**dadurch gekennzeichnet,**
**dass** das alkylsilylierte Halogenid Trimethylsilylchlorid oder Trimethylchlorsilan ist.

5. Verfahren nach einem der vorhergehenden Ansprüche,
**dadurch gekennzeichnet,**
**dass** das organische Medium Acetonitril umfasst.

6. Verfahren nach einem der vorhergehenden Ansprüche,
**dadurch gekennzeichnet,**
**dass** das oder die Fettsäureester in (einem) Fettsäuretriglycerid(en) besteht(en), das (die) von (einem) pflanzlichen Öl(en) stammen, das (die) in der Gruppe, die von Rapsöl, Mohnöl, Sojaöl, Safloröl, Erdnussöl, Traubenkernöl, Sonnenblumenöl, Leinöl, Maisöl, Olivenöl, Sesamöl, Weizenkeimöl, Kokosnussöl, Palmöl und den Ölen tierischen Ursprungs gebildet ist, ausgewählt wird (werden).

7. Verfahren nach einem der Ansprüche 1 bis 5,
**dadurch gekennzeichnet,**
**dass** das oder die Fettsäureester in (einem) Fettsäuremethylester(n) besteht(en), das (die) von (einem) pflanzlichen Öl(en) stammt(en), das (die) in der Gruppe, die von Rapsöl, Mohnöl, Sojaöl, Safloröl, Erdnussöl, Traubenkernöl, Sonnenblumenöl, Leinöl, Maisöl, Olivenöl, Sesamöl, Weizenkeimöl, Kokosnussöl, Palmöl und den Ölen tierischen Ursprungs gebildet ist, ausgewählt wird (werden).

8. Verfahren nach Anspruch 7,
**dadurch gekennzeichnet,**
**dass** das oder die Fettsäureester in (einem) Fettsäuremethylester(n), das (die) von Rapsöl stammt(en) und als biologischer Treibstoff für Fahrzeugmotoren verwendet wird (werden), besteht(en).

9. Verfahren nach einem der Ansprüche 1 bis 5,
**dadurch gekennzeichnet,**
**dass** das oder die Fettsäuerester in (einem) Fettsäureethylester(n) besteht(en), das (die) von (einem) pflanzlichen Öl(en) stammt(en), das (die) in der Gruppe, die von Rapsöl, Mohnöl, Sojaöl, Safloröl, Erdnussöl, Traubenkernöl, Sonnenblumenöl, Leinöl, Maisöl, Olivenöl, Sesamöl, Weizenkeimöl, Kokosnussöl, Palmöl und den Ölen tierischen Ursprungs gebildet ist, ausgewählt wird (werden).

10. Verfahren nach einem der Ansprüche 1 bis 5,
**dadurch gekennzeichnet,**
**dass** die Fettsäure(n) in der Gruppe, die von der Oleinsäure, der Linolsäure, der α-Linolensäure, der Erucasäure, der Arachisölsäure und der Ricinolsäure gebildet ist, ausgewählt wird (werden).

11. Jodhaltige Fettsäure(n), jodhaltige(s) Fettsäureester oder ihre jodhaltigen Derivate, die durch das Verfahren nach einem der Ansprüche 1 bis 10 gewonnen werden und keinerlei Doppelbindung enthalten, für die Herstellung eines Medikaments, das für die Behandlung gewisser Krebsarten durch Chemoembolisierung bestimmt ist.

12. Jodhaltige Fettsäure(n), jodhaltige(s) Fettsäureester oder ihre jodhaltigen Derivate, die durch das Verfahren nach einem der Ansprüche 1 bis 10 gewonnen werden und keinerlei Doppelbindung enthalten, für die Herstellung von Antikrebsmitteln, die in der bzw. den jodierten Fettsäure(n), jodierten Fettsäureester(n) oder ihren jodierten Derivaten emulgiert werden, wobei letztgenannte(s) als Vektor(en) wirkt(en), um die lipophilen Tumorzellen zu erreichen.

## Claims

1. Process for obtaining at least one iodinated fatty acid or at least one iodinated fatty acid ester or iodinated derivatives thereof which have pharmaceutical purity, are stable and free from toxic impurities, **characterised in that** it involves reacting an alkaline iodide with an alkylsilylated reagent in an organic medium giving rise in the presence of water to hydroiodic acid in situ reacting with the fatty acid(s), the fatty acid ester(s) or derivatives thereof in such a way that all the double bonds initially present in the fatty acid(s) or fatty acid ester(s) or derivatives thereof are saturated in iodine in a proportion of one molecule of hydroiodic acid per double bond.

2. Process according to claim 1, **characterised in that** the alkaline iodide is sodium iodide.

3. Process according to claim 1 or 2, **characterised in that** the alkylsilylated reagent is an alkylsilylated halide.

4. Process according to claim 3, **characterised in that** the alkylsilylated halide is trimethylsilyl chloride or trimethylchlorosilane.

5. Process according to any one of the preceding claims, **characterised in that** the organic medium contains acetonitrile.

6. Process according to any one of the preceding claims, **characterised in that** the fatty acid ester(s) consist(s) of fatty acid triglyceride(s) originating from vegetable oil(s) selected from the group formed by rapeseed oil, poppy-seed oil, soya-oil, safflower oil, groundnut oil, grapeseed oil, sunflower oil, linseed oil, corn oil, olive oil, sesame oil, wheatgerm oil, coconut oil, palm oil and oils of animal origin.

7. Process according to any one of claims 1 to 5, **characterised in that** the fatty acid ester(s) consist(s) of fatty acid methyl ester(s) originating from vegetable oil(s) selected from the group formed by rapeseed oil, poppy-seed oil, soya-oil, safflower oil, groundnut oil, grapeseed oil, sunflower oil, linseed oil, corn oil, olive oil, sesame oil, wheatgerm oil, coconut oil, palm oil and oils of animal origin.

8. Process according to claim 7, **characterised in that** the fatty acid ester(s) consist(s) of fatty acid methyl ester(s) originating from rapeseed oil, used as biological fuel for car engines.

9. Process according to any one of claims 1 to 5, **characterised in that** the fatty acid ester(s) consist(s) of fatty acid ethyl ester(s) originating from vegetable oil(s) selected from the group formed by rapeseed oil, poppy-seed oil, soya-oil, safflower oil, groundnut oil, grapeseed oil, sunflower oil, linseed oil, corn oil, olive oil, sesame oil, wheatgerm oil, coconut oil, palm oil and oils of animal origin.

10. Process according to any one of claims 1 to 5, **characterised in that** the fatty acid(s) is/are selected from the group formed by oleic acid, linoleic acid, α-linolenic acid, erucic acid, arachidonic acid and ricinoleic acid.

11. Iodinated fatty acid(s), iodinated fatty acid ester(s) or iodinated derivatives thereof obtained by the process according to any one of claims 1 to 10, not containing a double bond, for manufacturing a drug intended for the treatment of some cancers by chemoembolisation.

12. Iodinated fatty acid(s), iodinated fatty acid ester(s) or iodinated derivatives thereof obtained by the process according to any one of claims 1 to 10, not containing a double bond, for manufacturing an anti-cancer drug emulsified in said iodinated fatty acid(s), iodinated fatty acid ester(s) or iodinated derivatives thereof, acting as vector(s) to target the lipophilic tumoral cells.
